# EUROPEAN PATENT SPECIFICATION

(11) **EP 3 500 232 B1**
(45) Date of publication and mention of the grant of the patent: **10.06.2020**
(21) Application number: 17748502.6
(22) Date of filing: 08.08.2017
(51) Int. Cl.: A61K 8/24, A61K 8/25, A61Q 11/00, A61K 8/23

(54) **ORAL CARE COMPOSITION**
MUNDPFLEGEZUSAMMENSETZUNG
COMPOSITION DE PROTECTION ORALE

(30) Priority: 19.08.2016 WO PCT/CN2016/000469; 23.09.2016 EP 16190387
(43) Date of publication of application: 26.06.2019
(73) Proprietor: Unilever N.V., 3013 AL Rotterdam (NL); Unilever PLC, London, Greater London EC4Y 0DY (GB)
(72) Inventor: LI, Xiaoke, Shanghai 200335 (CN); LIU, Weining, Shanghai 200335 (CN); XING, Huaiyong, Shanghai 200335 (CN)
(74) Representative: Tansley, Sally Elizabeth
(86) International application number: PCT/EP2017/070021
(87) International publication number: WO 2018/033427

(56) References cited:
- WO-A1-98/07448
- WO-A1-2008/068149
- WO-A1-2015/036277
- WO-A1-2015/176867
- WO-A1-2015/189041
- WO-A2-2008/015117
- WO-A2-2014/056713
- CN-A- 104 490 608
- US-A1- 2001 046 475
- US-A1- 2007 183 984

## Description

### Technical Field of the Invention

The present invention relates to oral care compositions such as tooth pastes, powders, gums, mouthwashes and the like. In particular the present invention relates to an oral care composition comprising calcium silicate, a potassium phosphate salt and a tubule blockage enhancer that results in reducing sensitivity and/or remineralization of teeth. The invention also relates to the use of such compositions for treating tooth hypersensitivity and/or remineralizing and/or whitening of teeth of an individual.

### Background of the Invention

Tooth hypersensitivity is a temporary induced pain sensation that affects up to 20% of the adult population. Hypersensitive teeth may be sensitive to temperature, pressure or chemical action.

The dentin of the tooth generally contains channels, called tubules, which provide for an osmotic flow between the inner pulp region of the tooth and the outer root surfaces. Tooth hypersensitivity may be related to the general increase in exposed root surfaces of teeth as a result of periodontal disease, toothbrush abrasion, or cyclic loading fatigue of the thin enamel near the dentin-enamel junction. When root surfaces are exposed, dentinal tubules are also exposed.

The currently accepted theory for tooth hypersensitivity is the hydrodynamic theory, based on the belief that open exposed dentinal tubules allow fluid flow through the tubules. This flow excites the nerve endings in the dental pulp. Clinical replica of sensitive teeth viewed in a SEM (scanning electron microscopy) reveal varying numbers of open or partially occluded dentinal tubules.

There are different approaches to treat tooth hypersensitivity. One approach is to reduce the excitability of the nerve in a sensitive tooth by using "nerve-depolarising agents" comprising strontium ions, potassium salts such as potassium nitrate, potassium bicarbonate, potassium chloride and the like. These nerve-depolarising agents function by interfering with neural transduction of the pain stimulus to make the nerve less sensitive.

Another approach is to use "tubule blocking agents" that fully or partially occlude tubules such as polystyrene beads, apatite, polyacrylic acid, mineral hectorite clay and the like. These tubule blocking agents function by physically blocking the exposed ends of the dentinal tubules, thereby reducing dentinal fluid movement and reducing the irritation associated with the shear stress described by the hydrodynamic theory.

An ideal tubule blocking agent should result in faster and long lasting tubule occlusion. Tubule occlusion can be achieved either by deposition of mineral crystals on dentine surfaces and/or within the dentinal tubules. Superficial deposition of mineral crystals on dentine surfaces can provide short-term relief but the precipitate can be removed either by daily brushing and/or dissolved by saliva and/or consumption of acidic beverages. Occlusion within the dentinal tubules provides effective treatment with long-term benefit.

There is a continuing need for treating tooth hypersensitivity with a more efficient approach. The present inventors have now found unexpectedly that an oral care composition comprising calcium silicate, a potassium phosphate salt and a tubule blockage enhancer provides excellent tubule blockage efficacy to reduce tooth sensitivity. Additionally, such a composition can also enhance the tooth remineralization efficacy and/or the deposition of particulate tooth whitening agent on tooth surfaces to provide tooth whitening benefit.

### Additional Information

WO 2008/068149 A (Unilever) discloses an oral care product comprising a first composition comprising an insoluble calcium salt that is not a calcium phosphate salt, a second independent composition comprising a source of phosphate ions, and a means for delivering each of the compositions to the surface of the teeth. The preferred insoluble calcium salt is calcium silicate.

WO 2015/189041 A1 (Unilever) discloses an oral care composition comprising a first composition comprising greater than 20 wt% of the first composition of a calcium salt, and a second aqueous composition comprising greater than 25 wt% of the total second composition of a potassium phosphate salt.

WO 2014/056713 A2 (Unilever) discloses an oral care composition comprising calcium silicate hydrate comprising from 20 to 70% SiO₂ by weight of the calcium silicate hydrate and physiologically acceptable carrier comprising humectant, surfactant, thickener or a mixture thereof.

CN 104490608 A (Univ Zhejiang) discloses a multiple composition for oral treatment. The multiple composition comprises 2-25 parts of bioglass and 0.1-10 parts of phosphate of potassium.

The publication above does not describe an oral care composition comprising calcium silicate, from 1 to 20% by weight of a potassium phosphate salt and a tubule blockage enhancer of calcium dihydrogen phosphate, wherein the calcium silicate and the potassium phosphate salt is present in a weight ratio from 10:1 to 1:5, and especially such oral care composition can treat tooth hypersensitivity and/or enhance tooth remineralization efficacy.

### Tests and Definitions

### Dentifrice

"Dentifrice" for the purposes of the present invention means a paste, powder, liquid, gum or other preparation for cleaning the teeth or other surfaces in the oral cavity.

### Tooth Paste

"Tooth paste" for the purpose of the present invention means a paste or gel dentifrice for use with a toothbrush. Especially preferred are tooth pastes suitable for cleaning teeth by brushing for about two minutes.

### Particle Size

"Particle size" for the purpose of the present invention means D50 particle size. The D50 particle size of a particulate material is the particle size diameter at which 50 wt% of the particles are larger in diameter and 50 wt% are smaller in diameter. For the purpose of the present invention, particle sizes and distribution are measured using Malvern Mastersizer 2000 and Malvern ZetaSizer Nano series.

### Composite Particle

"Composite particle" for the purpose of the present invention means a particle comprising a first component core and a second component coating wherein the core and coating are composed of different materials.

### Refractive Index

Refractive index is quoted at a temperature of 25°C and a wavelength of 589 nm.

### Tubule blockage enhancer

"Tubule blockage enhancer" for the purpose of the present invention means a material which aids deposition of actives such as calcium silicate and/or other benefit agents from the continuous phase of the composition onto dentine surfaces and/or into dentinal tubules to induce *in situ* generation of calcium phosphate that occludes the dentinal tubules and/or their exposed open ends. Benefit agents as used herein means an active typically delivered to human teeth and/or the oral cavity including the gums to enhance or improve a characteristic of those dental tissues.

### pH

pH is quoted at atmospheric pressure and a temperature of 25°C. When referring to the pH of an oral care composition, this means the pH measured when 5 parts by weight of the composition is uniformly dispersed and/or dissolved in 20 parts by weight pure water at 25°C. In particular the pH may be measured by manually mixing 5 g oral care composition with 20 mL water for 30 s, then immediately testing the pH with indicator or a pH meter.

### Solubility

"Soluble" and "insoluble" for the purpose of the present invention means the solubility of a source (e.g., like calcium salts) in water at 25°C and atmospheric pressure. "Soluble" means a source that dissolves in water to give a solution with a concentration of at least 0.1 moles per litre. "Insoluble" means a source that dissolves in water to give a solution with a concentration of less than 0.001 moles per litre. "Sparingly soluble", therefore, is defined to mean a source that dissolves in water to give a solution with a concentration of greater than 0.001 moles per litre and less than 0.1 moles per litre.

### Water of Hydration

"Water of hydration" for the purpose of the present invention means water chemically combined with a substance in a way that it can be removed by heating without substantially changing the chemical composition of the substance. In particular, water which could only be removed when heated above 200 °C. The water loss is measured using thermo gravimetric analysis (TGA) with a Netzsch TG instrument. The TGA is conducted under an N₂ atmosphere with heating rate of 10 degree/min in the range of 30 to 900 °C.

### Substantially Free

"Substantially free of" for the purpose of the present invention means less than 1.5%, and preferably less than 1.0%, and more preferably less than 0.75% and more preferably still less than 0.5%, and even more preferably less than 0.1% and most preferably from 0.0 to 0.01% by weight, based on total weight of the oral care composition, including all ranges subsumed therein.

### Dual-Phase

"Dual-Phase" for the purpose of the present invention means a composition having two independent phases which are physically separate.

### Anhydrous Composition

"Anhydrous composition" for the purpose of the present invention means the water content of the composition is less than 1.5%, preferably from 0.0 to 0.75% by total weight of the oral care composition.

### Viscosity

Viscosity of a tooth paste is the value taken at room temperature (25 °C) with a Brookfield Viscometer, Spindle No.4 and at a speed of 5 rpm. Values are quoted in centipoises (cP=mPa.s) unless otherwise specified.

### Remineralization

"Remineralization" for the purpose of the present invention means *in situ* (i.e. in the oral cavity) generation of calcium phosphate on teeth (including layers on teeth from 10 nm to 20 microns, and preferably from 75 nm to 10 microns, and most preferably, from 150 nm to 5 microns thick including all ranges subsumed therein) to reduce the likelihood of tooth sensitivity, tooth decay, regenerate enamel and/or improve the appearance of teeth by whitening through the generation of such new calcium phosphate.

### Miscellaneous

Except in the examples, or where otherwise explicitly indicated, all numbers in this description indicating amounts of material or conditions of reaction, physical properties of materials and/or use may optionally be understood as modified by the word "about".

All amounts are by weight of the final oral care composition, unless otherwise specified.

It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

### Summary of the Invention

In a first aspect, the present invention is directed to an oral care composition comprising:
a) calcium silicate;
b) from 1 to 20% by weight of a potassium phosphate salt;
c) a tubule blockage enhancer of calcium dihydrogen phosphate; and
d) a physiologically acceptable carrier;
wherein the calcium silicate and the potassium phosphate salt is present in a weight ratio from 10:1 to 1:5.

In a second aspect, the present invention is directed to a packaged oral care product comprising the oral care composition of the first aspect of this invention.

In a third aspect, the present invention is directed to a method of reducing sensitivity and/or remineralization and/or whitening of teeth of an individual comprising the step of applying the oral care composition of any embodiment of the first aspect to at least one surface of the teeth of the individual.

All other aspects of the present invention will more readily become apparent upon considering the detailed description and examples which follow.

### Detailed Description

Now it has been found that an oral care composition comprising calcium silicate, a potassium phosphate salt and a tubule blockage enhancer provides excellent tubule blockage efficacy to reduce tooth sensitivity. Additionally, such a composition can also enhance the tooth remineralization efficacy and/or the deposition of particulate tooth whitening agent on tooth surfaces to provide tooth whitening benefit.

### CALCIUM SILICATE

In a preferred embodiment, the calcium silicate used which has low water solubility and is made commercially available under the name Sorbosil CA40 by PQ Corporation. In another preferred embodiment, the calcium silicate is insoluble, present as the composite material calcium oxide-silica (CaO-SiO₂), which is described, for example, in international patent application published as WO 2008/01517(Unilever) which is hereby incorporated by reference in its entirety. For a calcium silicate composite material, the atom ratio of calcium to silicon (Ca:Si) may be from 1:30 to 5:1. The Ca:Si ratio is preferably 1:20 to 3:1, and more preferably, from 1:10 to 3:1, and most preferably, from about 1:7 to 3:1. The calcium silicate may comprise mono-calcium silicate, bi-calcium silicate, or tri-calcium silicate. The calcium silicate may be in a crystalline or amorphous state or even in a mesoporous state.

In addition to calcium oxide, silica, the particles comprising the calcium silicate which is not hydrated may comprise other components, such as metal cations, anions (such as phosphate) and the like. However, it is preferred that the particles comprise calcium oxide, silica in an amount of at least 70% by weight of the particles, more preferably at least 80%, more preferably still at least 90% and even more preferably at least 95%. Most preferably the particles consist of (or at least consist essentially of) calcium oxide, silica.

In another preferred embodiment, the calcium silicate is calcium silicate hydrate. The calcium silicate hydrate for use in the present invention comprises at least calcium oxide (CaO), silica (SiO₂) and water. Compared with conventional calcium silicate which are not hydrated, the calcium silicate hydrate comprises the water of hydration in an amount of at least 5% by weight of the calcium silicate hydrate, preferably at least 10%, more preferably at least 15%, even more preferably at least 20% and most preferably at least 25%. The water content is typically no greater than 50% by weight of the calcium silicate hydrate, more preferably no greater than 40%, even more preferably no greater than 35% and most preferably no greater than 30%.

The calcium silicate hydrate preferably comprises at least 20% silica by weight of the calcium silicate hydrate, more preferably at least 30%, more preferably still at least 40% and most preferably at least 55%. The silica content is preferably no greater than 70% by weight of the calcium silicate hydrate, more preferably no greater than 65% and most preferably no greater than 60%.

To provide calcium necessary for remineralization, the calcium silicate hydrate preferably comprises calcium oxide in an amount of at least 5% by weight of the calcium silicate hydrate, more preferably at least 7%, more preferably still at least 10%, even more preferably at least 12% and most preferably at least 15%. The calcium oxide content is typically no greater than 50% by weight of the calcium silicate hydrate, more preferably no greater than 40%, even more preferably no greater than 30% and most preferably no greater than 25%.

The calcium silicate hydrate preferably comprises Ca and Si in an atom ratio (Ca:Si) less than 3:1, more preferably less than 1:1, more preferably still from 1:1.5 to 1:4 and most preferably from 1:1.7 to 1:3.

The calcium silicate may be amorphous or at least partly crystalline or mesoporous. The calcium silicate is preferably particulate as this allows for maximum surface area for contact with dental tissue. Thus preferably the composition comprises particles comprising the calcium silicate. Preferably from 10 to 100%, and especially, from 25 to 100%, and most especially, from 70% to 100% by weight of the particles comprising calcium silicate used in this invention have a particle size from 100 nm to less than 50 microns, preferably from 500 nm to 30 microns, more preferably from 1 micron to 20 microns, most preferably from 1 micron to 10 microns.

In addition to calcium oxide, silica and water, the particles which comprise the calcium silicate hydrate may comprise other components, such as metal cations, anions (such as phosphate) and the like. However, it is preferred that the particles comprise CaO, SiO₂ and water in an amount of at least 70% by weight of the particles, more preferably at least 80%, more preferably still at least 90% and even more preferably at least 95%. Most preferably the particles consist of (or at least consist essentially of) CaO, SiO₂ and water.

Typically, the oral care composition of the present invention comprises from 1 to 80% by weight of the calcium silicate, more preferably from 3 to 50%, most preferably from 5 to 30%, based on the total weight of the oral care composition and including all ranges subsumed therein.

### POTASSIUM PHOSPHATE SALT

The potassium phosphate salt suitable for use in this invention is able to react with calcium silicate used to form hydroxyapatite *in situ.*

The potassium phosphate salt that may be used in this invention includes potassium dihydrogen phosphate, dipotassium hydrogen phosphate or mixtures thereof.

In one preferred embodiment, the potassium phosphate salt comprises or is potassium dihydrogen phosphate.

In another preferred embodiment, the potassium phosphate salt comprises or is a mixture of potassium dihydrogen phosphate and dipotassium hydrogen phosphate. The mixture preferably comprises the potassium dihydrogen phosphate and the dipotassium hydrogen phosphate in a weight ratio from 20:1 to 1:10, more preferably from 10:1 to 1:1. Preferably, the potassium phosphate salt results in an oral care composition having a pH from 4.0 to 10.0, more preferably from 5.0 to 8.0, and most preferably from 5.5 to 7.5. The oral care composition of the present invention comprises from 1 to 20% by weight of the potassium phosphate salt, preferably from 3 to 18%, most preferably from 5 to 10%, based on the total weight of the oral care composition and including all ranges subsumed therein.

The oral care composition comprises the calcium silicate and the potassium phosphate salt in a weight ratio from 10:1 to 1:5, preferably from 5:1 to 1:3, most preferably from 3:1 to 1:1.

### TUBULE BLOCKAGE ENHANCER

It has been discovered that the material used as tubule blockage enhancers in this invention is biocompatible, and undergoes rapid reaction with water and complete resorption onto the tooth surface, therefore it can be used to aid the deposition of oral care actives such as calcium silicate and/or other benefit agents onto the tooth surface.

The tubule blockage enhancer is calcium dihydrogen phosphate.

Typically, the oral care composition of the present invention comprises from 0.1 to 20% by weight of the tubule blockage enhancer, more preferably from 0.2 to 15%, more preferably still from 0.5 to 10%, most preferably from 1 to 5%, based on the total weight of the oral care composition and including all ranges subsumed therein.

The oral care composition preferably comprises the calcium silicate and the tubule blockage enhancer in a weight ratio from 20:1 to 1:5, more preferably from 15:1 to 1:3, most preferably from 10:1 to 1:1.

### CARRIER

The composition of the present invention is an oral care composition and typically comprises a physiologically acceptable carrier. The carrier preferably comprises at least surfactant, thickener, humectant or a combination thereof.

Preferably the oral care composition comprises a surfactant. Preferably the composition comprises at least 0.01% surfactant by weight of the composition, more preferably at least 0.1% and most preferably from 0.5 to 7%. Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. More preferably the surfactant comprises or is anionic surfactant. The preferred anionic surfactants are sodium lauryl sulphate and/or sodium dodecylbenzene sulfonate. Most preferably the surfactant is sodium lauryl sulphate, sodium coco sulfate, cocamidopropyl betaine or mixtures thereof.

Thickener may also be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum arabic, gum karaya, sodium alginate, carrageenan, guar, xanthan gum, Irish moss, starch, modified starch, silica based thickeners including silica aerogels, magnesium aluminum silicate (e.g., Veegum), Carbomers (cross-linked acrylates) and mixtures thereof.

Typically, xanthan gum and/or sodium carboxymethyl cellulose and/or a Carbomer is/are preferred. When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

In another especially preferred embodiment, the thickener is xanthan gum.

Thickener typically makes up from 0.01 to about 10%, more preferably from 0.1 to 9%, and most preferably, from 0.1 to 5% by weight of the oral care composition, based on total weight of the composition and including all ranges subsumed therein.
When the oral care composition of this invention is a toothpaste or gel, the same typically has a viscosity from about 30,000 to 180,000 centipoise, and preferably, from 60,000 to 170,000 centipoise, and most preferably, from 65,000 to 165,000 centipoise.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants. The humectant may be present in the range of from 10 to 90% by weight of the oral care composition. More preferably, the carrier humectant makes up from 25 to 80%, and most preferably, from 30 to 60% by weight of the composition, based on total weight of the composition and including all ranges subsumed therein.

### OPTIONAL COMPONENTS

The oral care composition may further comprise benefit agents that are typically delivered to human teeth and/or the oral cavity including the gums to enhance or improve a characteristic of those dental tissues. The only limitation with respect to the benefit agents that may be used in this invention is that the same is suitable for use in the mouth. Typically the benefit agents include coloring agents, biomineralization agents, antibacterial agents or mixtures thereof. For example, coloring agent such as particulate whitening agents and pigments, preferably a particulate whitening agent. Preferably, the pigment, when used, is violet or blue having a hue angle, h, in the CIELAB system of from 220 to 320 degrees. These pigments may be selected from one or more of those listed in the Colour Index International, listed as pigment blue 1 through to pigment blue 83, and pigment violet 1 through to pigment violet 56; biomineralization agents for tooth enamel remineralization may be one or more of amorphous calcium phosphate, α-tricalcium phosphate, β-tricalcium phosphate; calcium carbonate, calcium deficient hydroxyapatite (Ca₉(HPO₄)(PO₄)₅OH), dicalcium phosphate (CaHPO₄), dicalcium phosphate dehydrate (CaHPO₄ • 2H₂O), hydroxyapatite (Ca₁₀(PO₄)₆(OH)₂), monocalcium phosphate monohydrate (Ca(H₂PO₄)₂ • H₂O), octacalcium phosphate (Ca₈H₂(PO₄)₆ • 5H₂O) and tetracalcium phosphate (Ca₄(PO₄)₂O); antibacterial agents may be selected from one or more of metal salts where the metal is selected from zinc, copper, silver or a mixture thereof, triclosan, triclosan monophosphate, triclocarban, curcumin, quaternary ammonium compounds, bisbiguanides and long chain tertiary amines, preferably zinc salts including zinc oxide, zinc chloride, zinc acetate, zinc ascorbate, zinc sulphate, zinc nitrate, zinc citrate, zinc lactate, zinc peroxide, zinc fluoride, zinc ammonium sulfate, zinc bromide, zinc iodide, zinc gluconate, zinc tartarate, zinc succinate, zinc formate, zinc phenol sulfonate, zinc salicylate, zinc glycerophosphate or a mixture thereof.

The benefit agent is preferably particulate as this allows for maximum surface area for contact with dental tissue.

In a preferred embodiment, the benefit agent is a particulate whitening agent for tooth whitening.

Typically, the particulate whitening agent comprises a material suitable to physically and immediately improve characteristics of teeth and especially whiten teeth. In order to provide excellent whitening effect, the material is preferred to have a high refractive index of at least 1.9, more preferably at least 2.0, even more preferably at least 2.2, even more preferably still at least 2.4 and most preferably at least 2.5. The maximum refractive index of the material is not particularly limited but preferably up to 4.0. Preferably, the material has a refractive index ranging from 1.9 to 4.0.

Particularly suitable materials are metal compounds and preferred are compounds where the metal is selected from zinc (Zn), titanium (Ti), zirconium (Zr) or a combination thereof. Preferably, the metal compound is (or at least comprises) a metal oxide such as titanium dioxide (TiO₂), zinc oxide (ZnO), zirconium dioxide (ZrO₂) or a combination thereof. In addition, the particulate whitening agent can also comprise non-metal oxides such as silicon monoxide (SiO).

In a preferred embodiment, the particulate whitening agent comprises metal oxides, non-metal oxides or a combination thereof in an amount of at least 50% by weight of the whitening agent and more preferably at least 70%, more preferably still from 80 to 100% and most preferably from 85 to 95%. In an especially preferred embodiment, the particulate whitening agent is at least 50% by weight titanium dioxide, and most preferably, from 60 to 100% by weight titanium dioxide, based on total weight of the whitening agent and including all ranges subsumed therein. In another especially preferred embodiment, the particulate whitening agents are slightly soluble or insoluble in water, but most preferably, insoluble in water.

In a preferred embodiment, the particulate whitening agents are composite particles. The refractive index of a composite particle comprising more than one material can be calculated based on the refractive indices and volume fractions of the constituents using effective medium theory, as is described for example in WO 2009/023353.

The composite particle comprises a first component core and a second component coating. Typically, the core of the composite particle comprises a material suitable to physically and immediately improve characteristics of teeth and especially whiten teeth. In order to provide excellent whitening effect, the material is preferred to have a high refractive index of at least 1.9, more preferably at least 2.0, even more preferably at least 2.2, even more preferably still at least 2.4 and most preferably at least 2.5. The maximum refractive index of the material is not particularly limited but preferably up to 4.0. Preferably, the material has a refractive index ranging from 1.9 to 4.0.

Particular suitable materials are metal compounds and preferred are compounds where the metal is selected from zinc (Zn), titanium (Ti), zirconium (Zr) or a combination thereof. Preferably, the metal compound is (or at least comprises) a metal oxide such as titanium dioxide (TiO₂), zinc oxide (ZnO), zirconium dioxide (ZrO₂) or a combination thereof. In addition, the core of the composite particle can also comprise non-metal oxides such as silicon monoxide (SiO).

The core of the composite particle typically makes up from 3 to 98%, and preferably from 6 to 65%, and most preferably from 10 to 55% by weight of the composite particle, based on total weight of the composite particle and including all ranges subsumed therein. In a preferred embodiment, the core comprises metal oxides, non-metal oxides or a combination thereof in an amount of at least 50% by weight of the core and more preferably at least 70%, more preferably still from 80 to 100% and most preferably from 85 to 95%. In an especially preferred embodiment, the core is at least 50% by weight titanium dioxide, and most preferably, from 60 to 100% by weight titanium dioxide, based on total weight of the first component core.

The second component coating comprises material suitable to adhere to tooth enamel, dentin or both. Typically the coating material comprises the element calcium, and optionally, other metals like potassium, sodium, aluminium, magnesium as well as mixtures thereof whereby such optional metals are provided as, for example, sulphates, lactates, oxides, carbonates or silicates. Optionally, the coating material may be aluminium oxide or silica. In a preferred embodiment, the coating material is suitable to provide a biological or chemical improvement to teeth which is long term (e.g., results in hydroxyapatite formation). Preferably, the coating employed comprises at least 50% by weight elemental calcium, and most preferably, at least 65% by weight elemental calcium based on total weight of metal in the coating. In an especially preferred embodiment, the metal in the coating is from 80 to 100% by weight of elemental calcium, based on total weight of metal in the second component coating and including all ranges subsumed therein. In another especially preferred embodiment, the core and the coating are slightly soluble or insoluble in water, but most preferably, insoluble in water.

In an especially desired embodiment, the second component coating can comprise, for example, calcium phosphate, calcium gluconate, calcium oxide, calcium lactate, calcium carbonate, calcium hydroxide, calcium sulphate, calcium carboxymethyl cellulose, calcium alginate, calcium salts of citric acid, calcium silicate, mixture thereof or the like. In another desired embodiment, the calcium source in the coating comprises calcium silicate.

In yet another preferred embodiment, the coating can comprise the element calcium which originates from insoluble calcium silicate, present as the composite material calcium oxide-silica (CaO-SiO₂) as described in international patent applications published as WO 2008/015117 and WO 2008/068248.

When a calcium silicate composite material is employed as coating, the ratio of calcium to silicon (Ca:Si) may be from 1:10 to 3:1. The Ca:Si ratio is preferably from 1:5 to 3:1, and more preferably, from 1:3 to 3:1, and most preferably, from about 1:2 to 3:1. The calcium silicate may comprise mono-calcium silicate, bi-calcium silicate, or tri-calcium silicate whereby ratios of calcium to silicon (Ca:Si) should be understood to be atom ratios.

Usually, at least 30% of the outer surface area of the first component core is coated with the second component coating, preferably at least 50% of the core is coated with the coating, most preferably, from 70 to 100% of the outer surface area of the first component core is coated with the second component coating.

In an especially preferred embodiment, the particulate whitening agent is titanium dioxide coated with calcium silicate.

The particulate whitening agent according to the present invention can be of different sizes and shapes. The particles may be of spherical, platelet or irregular shape form. The diameter of the particulate whitening agent is often from 10 nm to less than 50 microns, and preferably, from 75 nm to less than 10 microns. In an especially preferred embodiment, the diameter of particles is from 100 nm to 5 microns, including all ranges subsumed therein. For composite particles, in a preferred embodiment, at least 40%, and preferably, at least 60%, and most preferably, from 75 to 99.5% of the diameter of the composite particle is the core, including all ranges subsumed therein.

The oral care composition of the present invention may comprise a single benefit agent or a mixture of two or more benefit agents. Typically, the benefit agent is present in an amount from 0.25 to 60%, and more preferably, from 0.5 to 40%, and most preferably, from 1 to 30% by total weight of the oral care composition and including all ranges subsumed therein.

When the benefit agents is incorporated into the oral care composition, the relative weight ratio of calcium silicate to benefit agent may range from 1:10 to 5:1, preferably from 1:5 to 3:1, most preferably from 1:3 to 1:1.

The oral care composition of the present invention is found to be effective in blocking dentinal tubules to reduce tooth sensitivity and inducing new hydroxyapatite layer formation on enamel surfaces. Without wishing to be bound by theory the present inventors believe that this may be because the calcium silicate in the oral care composition of the present invention reacts with the potassium phosphate salt to form calcium phosphate and/or Si-OH groups of the calcium silicate may have an affinity for Ca ions in teeth. The tubule blockage enhancer of the present invention may further enhance the deposition of calcium silicate and/or calcium phosphate precipitate on surfaces of dentine and/or enamel, which further enhances the tubule blockage efficacy and/or tooth remineralization efficacy. When benefit agents are present in the oral care composition, the remineralization of calcium silicate around the benefit agents further helps the retention of those benefit agents on tooth surfaces by enhancing their resistance to shear force.

The present inventors have found unexpectedly that potassium phosphate salts and sodium phosphate salts have different effect on the nucleation rate of calcium phosphate. It has been found that potassium ions may inhibit the rapid calcium phosphate formation on dentine surfaces, which allows calcium ions and phosphate ions to penetrate deeply into dentinal tubules, nucleate and deposit inside the tubules.

The oral care composition of the present invention may comprise a phosphate source in addition to the potassium phosphate salt which is included in the composition. Such phosphate source includes, for example, trisodium phosphate, monosodium dihydrogen phosphate, disodium hydrogen phosphate, sodium pyrophosphate, tetrasodium pyrophosphate, sodium hexametaphosphate, mixtures thereof or the like.

The oral care composition of the present invention may contain a variety of other ingredients which are common in the art to enhance physical properties and performance. These ingredients include antimicrobial agents, anti-inflammatory agents, anti-caries agents, plaque buffers, fluoride sources, vitamins, plant extracts, desensitizing agents, anti-calculus agents, biomolecules, flavors, proteinaceous materials, preservatives, opacifying agents, coloring agents, pH-adjusting agents, sweetening agents, particulate abrasive materials, polymeric compounds, buffers and salts to buffer the pH and ionic strength of the compositions, and mixtures thereof. Such ingredients typically and collectively make-up less than 20% by weight of the composition, and preferably, from 0.0 to 15% by weight, and most preferably, from 0.01 to 12% by weight of the composition, including all ranges subsumed therein.

The oral care composition of this invention can be used in a method of reducing sensitivity and/or remineralizing and/or whitening of teeth of an individual comprising applying the composition to at least one surface of the teeth of an individual. The oral care composition of this invention may additionally or alternatively be for use as a medicament and/or used in the manufacture of a medicament for providing an oral care benefit as described herein, such as for reducing sensitivity of the teeth of an individual. Alternatively and preferably, the use is non-therapeutic.

In a preferred embodiment, the oral care composition is a monophase anhydrous composition. The composition is substantially free of water to prevent the premature reaction between the calcium silicate and the potassium phosphate salts.

In another preferred embodiment, the oral care composition is a dual-phase composition comprising a calcium phase and a phosphate phase, wherein the calcium silicate and the tubule blockage enhancer are present in the calcium phase and the potassium phosphate salt is present in the phosphate phase. The two phases are physically separate from one another by being in independent phases. The delivery of the two independent phases to the teeth may be simultaneous or sequential. In a preferred embodiment, the phases are delivered simultaneously.

Typically, the dual-phase composition is delivered by a dual-tube having a first compartment for calcium phase and a second compartment for phosphate phase, which allows for co-extrusion of the two phases.

In a preferred embodiment, such a dual-tube has one of the compartments surrounding the other. In such embodiments, one phase is present as a sheath, surrounding the other phase in the core. In an especially preferred embodiment, the core is the calcium phase and the sheath is the phosphate phase.

In another preferred embodiment, such a dual-tube has the two compartments side by side within the same tube. In such embodiments, the two phases are extruded from the tube as one, such extrusion being termed "contact extrusion". A pump head may be used in such a dual-tube for squeezing the two phases from the tube as one.

The dual-phase oral care composition may be a gel composition that comprises two independent gel phases, the first is the calcium phase and the second is the phosphate phase. The means of delivery may involve a cotton rod, or a tray, onto which the calcium phase and the phosphate phase are applied, prior to the tray being placed in contact with the teeth.

Typically the composition will be packaged. In tooth paste or gel form, the composition may be packaged in a conventional plastic laminate, metal tube or a single compartment dispenser. The same may be applied to dental surfaces by any physical means, such as a toothbrush, fingertip or by an applicator directly to the sensitive area.

The composition can be effective even when used in an individual's daily oral hygiene routine. For example, the composition may be brushed onto the teeth. The composition may, for example, be contacted with the teeth for a time period of one second to 20 hours. More preferably from 1 s to 10 hours, more preferably still from 10 s to 1 hour and most preferably from 30 s to 5 minutes. The composition may be used daily, for example for use by an individual once, twice or three times per day. When the oral care composition is a dual-phase composition, the two phases of the composition are mixed during application. The mixed phases are typically left on the teeth for from 3 minutes to 10 hours, more preferably from 3 minutes to 8 hours. The application may be carried out one to five times monthly.

The following examples are provided to facilitate an understanding of the present invention. The examples are not provided to limit the scope of the claims.

### Examples

### Example 1

This example demonstrates the improved blockage of dentinal tubules by using calcium silicate, potassium phosphate salt and a tubule blockage enhancer of calcium dihydrogen phosphate. All ingredients are expressed by weight percent of the total formulation, and as level of active ingredient.

**TABLE 1**

| Ingredient | Samples | | | |
|---|---|---|---|---|
| | **1** | **2** | **3** | **4** |
| Glycerin | 66.84 | 64.84 | 66.84 | 64.84 |
| Sodium monofluorophosphate | 1.11 | 1.11 | 1.11 | 1.11 |
| Sodium saccharin | 0.25 | 0.25 | 0.25 | 0.25 |
| Calcium silicate^{a} | 15.00 | 15.00 | 15.00 | 15.00 |
| Calcium dihydrogen phosphate | -- | 2.00 | -- | 2.00 |
| Sodium lauryl sulfate | 2.00 | 2.00 | 2.00 | 2.00 |
| Monosodium dihydrogen phosphate | 3.20 | 3.20 | -- | -- |
| Trisodium phosphate | 3.80 | 3.80 | -- | -- |
| Potassium dihydrogen phosphate | -- | -- | 6.00 | 6.00 |
| Dipotassium hydrogen phosphate | -- | -- | 1.00 | 1.00 |
| Silica abrasive^{b} | 6.00 | 6.00 | 6.00 | 6.00 |
| Silica abrasive^{c} | 0.50 | 0.50 | 0.50 | 0.50 |
| Xanthan gum | 0.10 | 0.10 | 0.10 | 0.10 |
| flavor | 1.20 | 1.20 | 1.20 | 1.20 |

| | | | | |
|---|---|---|---|---|
| a. Commercially available calcium silicate (CaSiO₃) under the trade name Sorbosil CA40 from PQ Corporation. b. Commercially available silica under the trade name Sorbosil AC77 from PQ Corporation. c. Commercially available silica under the trade name Sorbosil AC43 from PQ Corporation. | | | | |

### Methods

To evaluate the blockage efficacy of dentinal tubules, the test sample was mixed with water at a ratio of 4 g to 8 mL water to make a slurry.

Human dentine disks were eroded by 37% phosphoric acid for 1 min, then they were treated with different slurries via brushing following the same protocol. The dentine disks were brushed with the slurry under a tooth brushing machine equipped with toothbrushes. The load of the tooth brushing was 170 g +/-5 g and the automatic brushing operated at a speed of 150 rpm. After brushing for 1 min, the dentine disks were soaked in toothpaste slurry for 1 min. Then the dentine disks were rinsed with distilled water and placed in simulated oral fluid (SOF) under the condition of a shaking water bath at 37°C and 60.0 rpm. After soaking for about 3 to 4 hours, the dentine disks were brushed with the slurry by machine using the same procedure as in the first step. The brushing was repeated three times for one day, then the dentine disks were kept in SOF overnight(>12 hours) in a shaking water bath at 37°C to mimic oral environment. The dentine samples were brushed for 7 and 14 times.

Simulated oral fluid was made by combining the ingredients in Table 2:

**TABLE 2**

| Ingredient | Amount/g |
|---|---|
| NaCl | 16.07 |
| NaHCO₃ | 0.7 |
| KCl | 0.448 |
| K₂HPO₄*3H₂O | 3.27 |
| MgCl₂*6H₂O | 0.0622 |
| 1M HCl | 40 mL |
| CaCl₂ | 0.1998 |
| Na₂SO₄ | 0.1434 |
| Buffer | Adjust pH to 7.0 |
| Water | Balance to 2 L |

### Results

After 7 brushings, SEM (scanning Electron Microscopy) images of the dentine disks were taken. From the top view of the SEM images, it can be seen that Samples 3 comprising potassium phosphate salts showed better tubule blockage efficacy compared to Sample 1 comprising sodium phosphate salts. Samples 2 and 4 which further comprised a tubule blockage enhancer showed even better tubule blockage efficacy than Sample 3.

After 14 brushings, from the top view of the SEM images, all the samples showed good deposition on dentine surfaces and the dentinal tubules were extensively blocked. But the corresponding cross-section SEM images after 14 brushings showed that for Samples 3 and 4 comprising potassium phosphate salts, the materials penetrated deeper into the dentinal tubules than Samples 1 and 2. The dentine disks treated with Samples 1 and 2 showed almost empty dentinal tubules with some occlusion near the dentine surfaces, while the dentine disks treated with Sample 3 showed partial occlusion within the dentinal tubules. Sample 4 showed very deep penetration, and the dentinal tubules were completely occluded. Analysis using EDX (Energy Dispersive X-ray Spectroscopy) identified the occlusion within the dentinal tubules comprised the elements of Ca, P, O and Si, indicating the calcium silicate deposited into the dentinal tubules and induced remineralization. From the SAED (selected area electron diffraction) pattern obtained by TEM (Transmission Electron Microscopy), it clearly showed that the occlusion within the dentinal tubules was hydroxyapatite (HAP), which indicated transformation of deposited calcium silicate into HAP.

### Example 2

This example demonstrates the effect of the potassium salts in the formulations on dentinal tubule blockage efficacy. All ingredients are expressed by weight percent of the total formulation, and as level of active ingredient.

**TABLE 3**

| **Ingredient** | **Samples** | |
|---|---|---|
| | **5** | **6** |
| Glycerin | 60.84 | 62.30 |
| Sodium monofluorophosphate | 1.11 | 1.11 |
| Sodium saccharin | 0.25 | 0.25 |
| Calcium silicate^{a} | 15.00 | 15.00 |
| Calcium dihydrogen phosphate | 2.00 | 2.00 |
| Sodium lauryl sulfate | 2.00 | 2.00 |
| Monosodium dihydrogen phosphate | 6.00 | 6.00 |
| Potassium nitrate (KNO₃) | 5.00 | -- |
| Potassium carbonate (K₂CO₃) | -- | 3.54 |
| Silica abrasive^{b} | 6.00 | 6.00 |
| Silica abrasive^{c} | 0.50 | 0.50 |
| Xanthan gum | 0.10 | 0.10 |
| flavor | 1.20 | 1.20 |

### Methods

The same protocol was used to evaluate the blockage efficacy of dentinal tubules as described in Example 1. The dentine samples were brushed for 14 times.

### Results

After 14 brushings, SEM images of the dentine disks were taken. From the top view of the SEM images, it can be seen that Samples 5 and 6 showed some deposition on dentine surfaces but lots of dentinal tubules were still open. The corresponding cross-section SEM images showed that the dentine disks treated with Samples 5 and 6 had partial occlusion within the dentinal tubules.

### Example 3

This example demonstrates the weight ratio of potassium dihydrogen phosphate and dipotassium hydrogen phosphate can affect the tubule blockage efficacy. All ingredients are expressed by weight percent of the total formulation, and as level of active ingredient.

**TABLE 4**

| Ingredient | Samples | | | | | |
|---|---|---|---|---|---|---|
| | **4** | **7** | **8** | **9** | **10** | **11** |
| Glycerin | 64.84 | 64.84 | 64.84 | 64.84 | 64.84 | 64.84 |
| Sodium monofluorophosphate | 1.11 | 1.11 | 1.11 | 1.11 | 1.11 | 1.11 |
| Sodium saccharin | 0.25 | 0.25 | 0.25 | 0.25 | 0.25 | 0.25 |
| Calcium silicate^{a} | 15.00 | 15.00 | 15.00 | 15.00 | 15.00 | 15.00 |
| Calcium dihydrogen phosphate | 2.00 | 2.00 | 2.00 | 2.00 | 2.00 | 2.00 |
| Sodium lauryl sulfate | 2.00 | 2.00 | 2.00 | 2.00 | 2.00 | 2.00 |
| Potassium dihydrogen phosphate | 6.00 | 7.00 | 4.00 | 2.00 | -- | -- |
| Dipotassium hydrogen phosphate | 1.00 | -- | 3.00 | 5.00 | 7.00 | -- |
| Tripotassium phosphate | -- | -- | -- | -- | -- | 7.00 |
| Silica abrasive^{b} | 6.00 | 6.00 | 6.00 | 6.00 | 6.00 | 6.00 |
| Silica abrasive^{c} | 0.50 | 0.50 | 0.50 | 0.50 | 0.50 | 0.50 |
| Xanthan gum | 0.10 | 0.10 | 0.10 | 0.10 | 0.10 | 0.10 |
| flavor | 1.20 | 1.20 | 1.20 | 1.20 | 1.20 | 1.20 |

### Methods

The same protocol was used to evaluate the blockage efficacy of dentinal tubules as described in Example 1. The dentine samples were brushed for 14 times.

### Results

After 14 brushings, SEM images of the dentine disks were taken. From the top view of the SEM images, it can be seen that all the samples except for Sample 11 showed good deposition on dentine surfaces and the dentinal tubules were extensively blocked. But for Sample 11 comprising tripotassium phosphate, lots of dentinal tubules were still open. The corresponding cross-section SEM images showed that all the samples except for Sample 11 showed deep penetration into the dentinal tubules. Particularly, Samples 4 and 7 showed better tubule blockage efficacy than other samples, the dentinal tubules were almost completely occluded.

### Example 4

This example demonstrates the improved deposition on tooth surfaces by using calcium silicate, potassium phosphate salt and a tubule blockage enhancer. All ingredients are expressed by weight percent of the total formulation, and as level of active ingredient.

**TABLE 5**

| **Ingredient** | **Samples** | |
|---|---|---|
| | **12** | **13** |
| Glycerin | 64.94 | 64.94 |
| Sodium monofluorophosphate | 1.11 | 1.11 |
| Sodium saccharin | 0.25 | 0.25 |
| Calcium silicate^{a} | 15.00 | 15.00 |
| Calcium dihydrogen phosphate | 2.00 | 2.00 |
| Sodium lauryl sulfate | 2.00 | 2.00 |
| Monosodium dihydrogen phosphate | 3.20 | -- |
| Trisodium phosphate | 3.80 | -- |
| Potassium dihydrogen phosphate | -- | 6.00 |
| Dipotassium hydrogen phosphate | -- | 1.00 |
| Silica abrasive^{b} | 6.00 | 6.00 |
| Silica abrasive^{c} | 0.50 | 0.50 |
| Xanthan gum | 0.10 | 0.10 |
| flavor | 1.10 | 1.10 |

### Methods

To evaluate deposition on tooth surfaces, the test sample was mixed with water at a ratio of 4 g to 8 mL water to make a toothpaste slurry.

The bovine enamel blocks were treated with different slurries via brushing following the same protocol. The enamel blocks were brushed with the slurry under a tooth brushing machine equipped with toothbrushes. The load of the tooth brushing was 170 g +/-5 g and the automatic brushing operated at a speed of 150 rpm. After brushing for 1 min, the enamel blocks were soaked in toothpaste slurry for 1 min. Then the enamel blocks were rinsed with distilled water and placed in SOF under the condition of a shaking water bath at 37°C and 150.0 rpm. After soaking for about 3 to 4 hours, the enamel blocks were brushed with the slurry by machine using the same procedure as in the first step. The brushing was repeated three times for one day, then the enamel blocks were kept in SOF overnight (>12 hours) in a shaking water bath at 37°C to mimic oral environment. The enamel blocks were brushed for 28 times.

### Results

After 28 brushings, SEM images of the enamel block surfaces were taken. From the top view of the SEM images, it showed that Sample 13 comprising potassium phosphate salts gave much better and denser deposition on enamel surfaces than Sample 12. The corresponding cross-section SEM images further showed that a much thicker new layer was formed on the enamel surfaces treated with Sample 13 than those treated with Sample 12. Analysis using EDX identified the elements of Ca and P within the new layer, indicating calcium silicate deposited on enamel surfaces and induced remineralization.

### Example 5

This example demonstrates the effect of calcium salts in the formulations on dentinal tubule blockage efficacy. All ingredients are expressed by weight percent of the total formulation, and as level of active ingredient.

**TABLE 6**

| **Ingredient** | **Samples** | | | |
|---|---|---|---|---|
| | **4** | **14** | **15** | **16** |
| Glycerin | 64.84 | 64.84 | 64.84 | 64.84 |
| Sodium monofluorophosphate | 1.11 | 1.11 | 1.11 | 1.11 |
| Sodium saccharin | 0.25 | 0.25 | 0.25 | 0.25 |
| Calcium silicate^{a} | 15.00 | 15.00 | 15.00 | 15.00 |
| Calcium dihydrogen phosphate | 2.00 | -- | -- | -- |
| Hydroxyapatite | -- | 2.00 | -- | -- |
| Calcium monohydrogen phosphate | -- | -- | 2.00 | -- |
| Calcium sulfate hemihydrate | -- | -- | -- | 2.00 |
| Sodium lauryl sulfate | 2.00 | 2.00 | 2.00 | 2.00 |
| Potassium dihydrogen phosphate | 6.00 | 6.00 | 6.00 | 6.00 |
| Dipotassium hydrogen phosphate | 1.00 | 1.00 | 1.00 | 1.00 |
| Silica abrasive^{b} | 6.00 | 6.00 | 6.00 | 6.00 |
| Silica abrasive^{c} | 0.50 | 0.50 | 0.50 | 0.50 |
| Xanthan gum | 0.10 | 0.10 | 0.10 | 0.10 |
| flavor | 1.20 | 1.20 | 1.20 | 1.20 |

### Methods

The same protocol was used to evaluate the blockage efficacy of dentinal tubules as described in Example 1. The dentine samples were brushed for 7 and 14 times.

### Results

After 7 brushings, SEM images of the dentine disks were taken. From the top view of the SEM images, it can be seen that Sample 4 comprising calcium dihydrogen phosphate showed the best tubule blockage efficacy compared to other samples. Sample 16 comprising calcium sulfate hemihydrate showed better tubule blockage efficacy than Samples 14 and 15 comprising hydroxyapatite and calcium monohydrogen phosphate respectively. Samples 14 and 15 showed some deposition on dentine surfaces but lots of dentinal tubules were still open.

The corresponding cross-section SEM images after 14 brushings showed that for Sample 4 comprising calcium dihydrogen phosphate, the materials penetrated deeper into the dentinal tubules than other samples. The dentine disks treated with Sample 14, 15 or 16 showed almost empty dentinal tubules with some occlusion near the dentine surfaces, while the dentine disks treated with Sample 4 showed very deep penetration, and the dentinal tubules were completely occluded.

## Claims

1. An oral care composition comprising:
a) calcium silicate;
b) from 1 to 20% by weight of a potassium phosphate salt;
c) a tubule blockage enhancer of calcium dihydrogen phosphate; and
d) a physiologically acceptable carrier;
wherein the calcium silicate and the potassium phosphate salt is present in a weight ratio from 10:1 to 1:5.

2. The oral care composition according to claim 1, wherein the potassium phosphate salt comprises potassium dihydrogen phosphate, dipotassium hydrogen phosphate or mixtures thereof.

3. The oral care composition according to claim 2, wherein the potassium phosphate salt comprises potassium dihydrogen phosphate.

4. The oral care composition according to claim 2, wherein the potassium phosphate salt comprises a mixture of potassium dihydrogen phosphate and dipotassium hydrogen phosphate.

5. The oral care composition according to claim 4, wherein the weight ratio of potassium dihydrogen phosphate and dipotassium hydrogen phosphate is from 20:1 to 1:10, preferably from 10:1 to 1:1.

6. The oral care composition according to any of the preceding claims, wherein the potassium phosphate salt is present in an amount from 3 to 18%, preferably from 5 to 10% by weight of the composition.

7. The oral care composition according to any of the preceding claims, wherein the calcium silicate is present in an amount from 0.1 to 50%, preferably 1 to 20% by weight of the composition.

8. The oral care composition according to any of the preceding claims, wherein the calcium silicate and the tubule blockage enhancer is present is a weight ratio from 20:1 to 1:5, preferably from 10:1 to 1:1.

9. The oral care composition according to any of the preceding claims, wherein the composition comprises the calcium silicate and the potassium phosphate salt in a weight ratio from 10:1 to 1:5, preferably from 5:1 to 1:3.

10. The oral care composition according to any of the preceding claims, wherein the composition further comprises a benefit agent, preferably a particulate whitening agent.

11. The oral care composition according to claim 10, wherein the particulate whitening agent is a composite particle, preferably the composite particle is titanium dioxide coated with calcium silicate.

12. The oral care composition according to any of the preceding claims, wherein the oral care composition is a monophase anhydrous composition.

13. The oral care composition according to any one of claims 1 to 11, wherein the composition is a dual-phase composition comprising a calcium phase and a phosphate phase, wherein the calcium silicate and the tubule blockage enhancer are present in the calcium phase, and the potassium phosphate salt is present in the phosphate phase.

14. The oral care composition according to claim 13, wherein the calcium phase and the phosphate phase are physically separate prior to the use of the composition.

15. An oral care composition according to any of the preceding claims for use in reducing sensitivity and/or remineralizing and/or whitening of the teeth of an individual comprising the step of applying the composition to at least one surface of the teeth of the individual.

## Patentansprüche

1. Mundpflegezusammensetzung, umfassend:
a) Calciumsilikat;
b) von 1 bis 20 Gewichts-% eines Kaliumphosphat-Salzes;
c) einen Tubulusblockade-Verbesserer von Calciumdihydrogenphosphat; und
d) einen physiologisch verträglichen Träger;
wobei das Calciumsilikat und das Kaliumphosphat-Salz in einem Gewichtsverhältnis von 10:1 bis 1:5 vorliegen.

2. Mundpflegezusammensetzung nach Anspruch 1, wobei das Kaliumphosphat-Salz Kaliumdihydrogenphosphat, Dikaliumhydrogenphosphat oder Mischungen davon umfasst.

3. Mundpflegezusammensetzung nach Anspruch 2, wobei das Kaliumphosphat-Salz Kaliumdihydrogenphosphat umfasst.

4. Mundpflegezusammensetzung nach Anspruch 2, wobei das Kaliumphosphat-Salz eine Mischung von Kaliumdihydrogenphosphat und Dikaliumhydrogenphosphat umfasst.

5. Mundpflegezusammensetzung nach Anspruch 4, wobei das Gewichtsverhältnis von Kaliumdihydrogenphosphat und Dikaliumhydrogenphosphat von 20:1 bis 1:10, vorzugsweise von 10:1 bis 1:1, beträgt.

6. Mundpflegezusammensetzung nach irgendeinem der vorhergehenden Ansprüche, wobei das Kaliumphosphat-Salz in einer Menge von 3 bis 18%, vorzugsweise von 5 bis 10%, bezogen auf das Gewicht der Zusammensetzung, vorliegt.

7. Mundpflegezusammensetzung nach irgendeinem der vorhergehenden Ansprüche, wobei das Calciumsilikat in einer Menge von 0,1 bis 50%, vorzugsweise von 1 bis 20%, bezogen auf das Gewicht der Zusammensetzung, vorliegt.

8. Mundpflegezusammensetzung nach irgendeinem der vorhergehenden Ansprüche, wobei das Calciumsilikat und der Tubulusblockade-Verbesserer in einem Gewichtsverhältnis von 20:1 bis 1:5, vorzugsweise von 10:1 bis 1:1, vorliegen.

9. Mundpflegezusammensetzung nach irgendeinem der vorhergehenden Ansprüche, wobei die Zusammensetzung das Calciumsilikat und das Kaliumphosphat-Salz in einem Gewichtsverhältnis von 10:1 bis 1:5, vorzugsweise von 5:1 bis 1:3, umfasst.

10. Mundpflegezusammensetzung nach irgendeinem der vorhergehenden Ansprüche, wobei die Zusammensetzung ferner ein Vorteilsmittel, vorzugsweise ein teilchenförmiges Aufhellungsmittel, umfasst.

11. Mundpflegezusammensetzung nach Anspruch 10, wobei das teilchenförmige Aufhellungsmittel ein Verbundpartikel ist, wobei das Verbundpartikel mit Calciumsilikat beschichtetes Titandioxid ist.

12. Mundpflegezusammensetzung nach irgendeinem der vorhergehenden Ansprüche, wobei die Mundpflegezusammensetzung eine wasserfreie Ein-Phasen-Zusammensetzung ist.

13. Mundpflegezusammensetzung nach irgendeinem der Ansprüche 1 bis 11, wobei die Zusammensetzung eine Zwei-Phasen-Zusammensetzung ist, die eine Calcium-Phase und eine Phosphat-Phase umfasst, wobei das Calciumsilikat und der Tubulusblockade-Verbesserer in der Calcium-Phase und das Kaliumphosphat-Salz in der Phosphat-Phase vorliegen.

14. Mundpflegezusammensetzung nach Anspruch 13, wobei die Calcium-Phase und die Phosphat-Phase vor der Verwendung der Zusammensetzung physikalisch getrennt vorliegen.

15. Mundpflegezusammensetzung nach irgendeinem der vorhergehenden Ansprüche zur Verwendung zum Reduzieren der Empfindlichkeit und/oder Remineralisieren und/oder Aufhellen der Zähne eines Individuums, umfassend den Schritt des Auftragens der Zusammensetzung auf mindestens eine Oberfläche der Zähne des Individuums.

## Revendications

1. Composition pour le soin oral comprenant :
a) du silicate de calcium ;
b) de 1 à 20 % en masse d'un sel de phosphate de potassium ;
c) un promoteur de blocage de tubule de dihydrogénophosphate de calcium ; et
d) un support physiologiquement acceptable ;
dans laquelle le silicate de calcium et le sel de phosphate de potassium sont présents dans un rapport de masse de 10:1 à 1:5.

2. Composition pour le soin oral selon la revendication 1, dans laquelle le sel de phosphate de potassium comprend du dihydrogénophosphate de potassium, hydrogénophosphate de dipotassium ou des mélanges de ceux-ci.

3. Composition pour le soin oral selon la revendication 2, dans laquelle le sel de phosphate de potassium comprend du dihydrogénophosphate de potassium.

4. Composition pour le soin oral selon la revendication 2, dans laquelle le sel de phosphate de potassium comprend un mélange de dihydrogénophosphate de potassium et hydrogénophosphate de dipotassium.

5. Composition pour le soin oral selon la revendication 4, dans laquelle le rapport massique de dihydrogénophosphate de potassium et d'hydrogénophosphate de dipotassium est de 20:1 à 1:10, de préférence de 10:1 à 1:1.

6. Composition pour le soin oral selon l'une quelconque des revendications précédentes, dans laquelle le sel de phosphate de potassium est présent dans une quantité de 3 à 18 %, de préférence de 5 à 10 % en masse de la composition.

7. Composition pour le soin oral selon l'une quelconque des revendications précédentes, dans laquelle le silicate de calcium est présent dans une quantité de 0,1 à 50 %, de préférence de 1 à 20 % en masse de la composition.

8. Composition pour le soin oral selon l'une quelconque des revendications précédentes, dans laquelle le silicate de calcium et le promoteur de blocage de tubule sont présents dans un rapport massique de 20:1 à 1:5, de préférence de 10:1 à 1:1.

9. Composition pour le soin oral selon l'une quelconque des revendications précédentes, dans laquelle la composition comprend le silicate de calcium et le sel de phosphate de potassium dans un rapport massique de 10:1 à 1:5, de préférence de 5:1 à 1:3.

10. Composition pour le soin oral selon l'une quelconque des revendications précédentes, dans laquelle la composition comprend de plus un agent bénéfique, de préférence un agent de blanchiment particulaire.

11. Composition pour le soin oral selon la revendication 10, dans laquelle l'agent de blanchiment particulaire est une particule composite, de préférence la particule composite est du dioxyde de titane revêtu de silicate de calcium.

12. Composition pour le soin oral selon l'une quelconque des revendications précédentes, dans laquelle la composition pour le soin oral est une composition anhydre monophase.

13. Composition pour le soin oral selon l'une quelconque des revendications 1 à 11, dans laquelle la composition est une composition à phase double comprenant une phase de calcium et une phase de phosphate, dans laquelle le silicate de calcium et le promoteur de blocage de tubule sont présents dans la phase de calcium, et le sel de phosphate de potassium est présent dans la phase de phosphate.

14. Composition pour le soin oral selon la revendication 13, dans laquelle la phase de calcium et la phase de phosphate sont physiquement séparées avant l'utilisation de la composition.

15. Composition pour le soin oral selon l'une quelconque des revendications précédentes pour une utilisation dans la réduction de la sensibilité et/ou la reminéralisation et/ou le blanchiment des dents d'un individu comprenant l'étape d'application de la composition sur au moins une surface des dents de l'individu.
